# EUROPÄISCHE PATENTANMELDUNG

(11) **EP 1 153 583 A1**
(43) Veröffentlichungstag der Anmeldung: **14.11.2001**
(21) Anmeldenummer: 01108441.5
(22) Anmeldetag: 04.04.2001
(51) Int. Cl.: A61F 9/007, A61F 9/011

(54) **Vorrichtung zur Glaukombehandlung**

(30) Priorität: 11.05.2000 DE 10023176
(71) Anmelder: Glautec AG, 90425 Nürnberg (DE)
(72) Erfinder: Haefliger, Eduard, Dr., 4067 Basel (CH)
(74) Vertreter: Matschkur, Peter

(57) **Zusammenfassung**

Vorrichtung zur Glaukombehandlung mit einem Laserkatheter (6) und einer lichtführenden Faseranordnung (7), an deren proximalem Ende Licht einkoppelbar ist und an deren distalem Ende eine Lichtaustrittsfläche vorgesehen ist, wobei am distalen Ende des Laserkatheters eine zur einfachen Ermittlung der richtigen Arbeitsposition bezüglich des Schlemm'schen Kanals (8) dienende Justiereinrichtung (10,13) vorgesehen ist.

## Beschreibung

Die Erfindung bezieht sich auf eine Vorrichtung zur Glaukombehandlung mit einem Laserkatheter und einer lichtführenden Faseranordnung, an deren proximalem Ende Licht einkoppelbar ist und an deren distalem Ende eine Lichtaustrittsfläche vorgesehen ist.

Zur Glaukombehandlung, also zur Beseitigung eines Überdrucks im Augapfel, gibt es neben medikamentösen Verfahren eine Reihe von chirurgischen Verfahren. Unter anderem ist dabei auch bereits eine Vorrichtung der vorstehend beschriebenen Art vorgeschlagen worden, bei welcher mittels UV-Licht das vorzugsweise über einen Excimerlaser erzeugt und über Lichtleiter in das Augeninnere geführt wird, das schwammartige Trabekelwerk, durch das Kammerwasser aus der vorderen und hinteren Augenkammer fließt, lokal abgetragen wird, so dass das Kammerwasser leichter in den Schlemm'schen Kanal gelangen kann, durch den es schließlich abgeführt wird.

Zum Einsatz dieser Vorrichtung ist es erforderlich, das Auge lokal zu öffnen, um mit Hilfe des lichtleitenden Laserkatheters Licht in unmittelbare Nähe des zu perforierenden Gewebes des Trabekelwerkes zu leiten. Notwendig ist es dabei, die Lichtaustrittsfläche genau vor dem Schlemm'schen Kanal zu positionieren, um genau an dieser Stelle das Trabekelwerk zu durchlöchern und den Abfluss in den Schlemm'schen Kanal sicherzustellen. Diese Positionierung des distalen Endes des Laserkatheters ist aber sehr schwierig und macht dadurch den Einsatz einer solchen Glaukombehandlungsvorrichtung bis zu einem gewissen Grad zu einem Glücksspiel, bei dem es darauf ankommt, ob man die richtige Position beim Einführen des Laserkatheters wirklich gefunden hat oder nicht.

Der Erfindung liegt daher die Aufgabe zugrunde, eine Vorrichtung der eingangs genannten Art so auszugestalten, dass in jedem Fall eine zwangsläufige "richtige" Positionierung der Lichtaustrittsfläche der Faseranordnung des Laserkatheters vor dem Schlemm'schen Kanal gewährleistet ist.

Zur Lösung dieser Aufgabe ist erfindungsgemäß vorgesehen, dass am distalen Ende des Laserkatheters eine zur einfachen Ermittlung der richtigen Arbeitsposition bezüglich des Schlemm'schen Kanals dienende Justiereinrichtung vorgesehen ist, wobei diese gemäß einer ersten Ausführungsform der vorliegenden Erfindung sehr einfach durch einen am distalen Ende des Laserkatheters vorgesehenen, zur Abstützung auf der Iriswurzel dienenden Justiernocken gebildet sein kann.

Ein derartiger Justiernocken, der bevorzugt an einem die Faseranordnung umgebenden Stützmantel angeformt sein kann, ermöglicht eine Benutzungsform der erfindungsgemäßen Vorrichtung derart, dass beim Anstoßen des distalen Endes des Laserkatheters einfach eine Kippbewegung nach unten erfolgt, so dass sich der Justiernocken auf der Iriswurzel abstützt. Durch entsprechende Ausbildung der Größe dieses Justiernockens ist dann gewährleistet, dass sich die Lichtaustrittsfläche der Faseranordnung genau vor dem Schlemm'schen Kanal befindet.

Gemäß einer zweiten Ausführungsform der Erfindung, wobei beide Ausführungsformen auch kombiniert an ein und dergleichen Vorrichtung vereint angewendet werden können - ist eine erfindungsgemäße Justiereinrichtung gekennzeichnet durch einen in den Schlemm'schen Kanal einführbaren Stent zur Ertastung der Arbeitsposition des Laserkatheters. Der Operateur verschwenkt nach dem Einsetzen des Laserkatheters in den Augapfel die Katheterspitze geringfügig und tastet dabei den Kammerwinkel ab. Sobald er auf den Stent trifft, vorzugsweise eine sogenannten Harms-Sonde nach Neuhann, die durch Lamellieren über einen Schnitt von außen in den Schlemm'schen Kanal einführbar ist, spürt er durch die Härte des Widerstandes des Stentes die gefundene richtige Arbeitsposition und kann dann durch Einschalten des Lasers die Durchlöcherung des Trabekelwerkes mittels der Laserstrahlung durchführen.

Mit besonderem Vorteil kann in Verbindung mit diesem Stent, bzw. der Harms-Sonde nach Neuhann, weiter vorgesehen sein, dass am distalen Ende des Laserkatheters eine zur Form des Stents komplementäre Justiereinrichtung vorgesehen ist, die beim Verkippen der Katheterspitze quasi auf die durch den Stent sich ergebende Ausbuchtung in den Kammerwinkel "aufschnappt" und damit die gefundene richtige Arbeitsposition dem Operateur zu erkennen gibt.

Die Justierausnehmung kann dabei wiederum bevorzugt in die Stirnfläche des Stützmantels der Faseranordnung eingeformt sein.

Schließlich liegt es auch noch im Rahmen der Erfindung, den Laserkatheters am distalen Ende mit einer dem Kammerwinkel des Auges angepassten Abschrägung zu versehen, so dass die Lichtaustrittsfläche möglichst parallel an den Schlemm'schen Kanal herangeführt werden kann.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: einen schematischen Querschnitt durch den Augapfel mit einem eingeführten Laserkatheter, und
- Fig. 2: eine vergrößerte Detailansicht des Kammerwinkels am distalen Ende eines erfindungsgemäß ausgestalteten Laserkatheters.

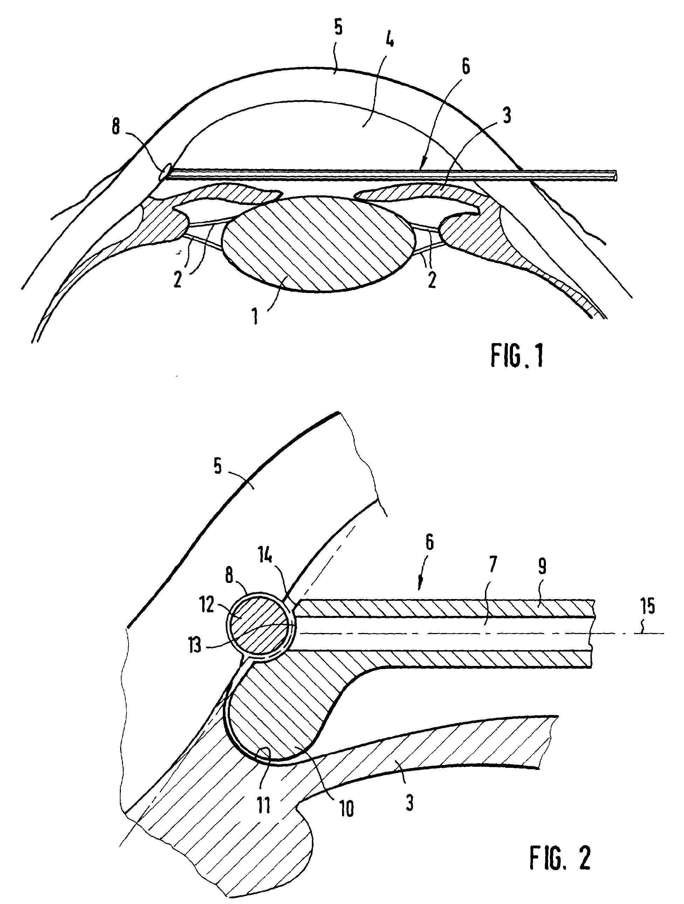

Die Fig. 1 zeigt einen Teilaquerschnitt durch den Augapfel eines Menschen. Mit 1 ist dabei die Linse, mit 2 die Zonulafasern zur Aufhängung der Linse, mit 3 die Iris, mit 4 die Pupille und mit 5 die Hornhaut bezeichnet. Bei 8 erkennt man den umlaufenden Schlemm'schen Kanal zur Abführung des Kammerwassers. Bei einer zu geringen Durchlässigkeit des vor dem Schlemm'schen Kanals liegenden nicht dargestellten Trabekelwerkes muss durch eine Durchlöcherung dieses Trabekelwerkes der Abfluss in den Schlemm'schen Kanal 8 wieder sichergestellt werden. Hierzu dient ein Laserkatheter 6, der erfindungsgemäß aus einer Faseranordnung 7 und einem diese umhüllenden Stützmantel 9 besteht. Am proximalen Ende dieser Lichtfaseranordnung ist ein Handgriff und ein Anschluss zu einem Excimerlaser od. dgl. vorgesehen. Diese Ausgestaltung ist an sich bekannt und braucht daher an dieser Stelle nicht näher beschrieben werden.

Erfindungsgemäß ist der Laserkatheter 6 am distalen Ende mit einer Justiereinrichtung versehen, die die exakte Positionierung der Lichtaustrittsfläche der Faseranordnung 7 genau vor dem Schlemm'schen Kanal 8 gewährleisten soll. Diese Justiereinrichtung kann dabei in verschiedener Weise ausgebildet sein, wobei in Fig. 2 beide möglichen Ausführungsformen gleichzeitig mit dargestellt sind.

Zum einen kann zur Justierung ein im dargestellten Ausführungsbeispiel am Stützmantel 9 der Faseranordnung 7 angeformter Justiernocken 10 vorgesehen sein, der sich auf der Iriswurzel 11 der Iris 3 abstützt. Nach Einführen des Laserkatheters kippt der Operateur die Spitze des Katheters leicht nach unten, so dass diese Abstützung stattfindet, wobei durch die Bemessung des Justiernockens 10 dann automatisch die Lichtaustrittsfläche der Faseranordnung 7 genau vor dem Schlemm'schen Kanal 8 liegt und damit das Trabekelwerk vor dem Schlemm'schen Kanal mittels der Strahlung durchlöchert werden kann.

Anstelle dieser Ausbildung der Justiervorrichtung als Justiernocken 10 - gegebenenfalls auch zusätzlich dazu - kann vorgesehen sein, dass in den Schlemm'schen Kanal 8 ein Stent 12, vorzugsweise in Form einer Harms-Sonde nach Neuhann, über einen Lamellierungsschnitt von außen eingeführt wird, so dass dieser harte Stent 12 vom Operateur durch Anstoßen mit der Spitze des Laserkatheters 6 ertastbar ist und damit die exakte Arbeitsposition gefunden werden kann. Im dargestellten Ausführungsbeispiel ist zusätzlich noch eine Justierausnehmung 13 am distalen Ende des Laserkatheters 6 vorgesehen, die komplementär der Form des Stents 12 entspricht, so dass die Spitze des Laserkatheters 6 quasi auf den in den Kammerwinkel gedrückten Stent "aufschnappt" und so die genaue Arbeitsposition der Faseranordnung 7 vor dem Schlemm'schen Kanal 8 sichert.

In an sich bekannter Weise kann bei dem erfindungsgemäßen Laserkatheter 6 am distalen Ende eine im Wesentlichen dem Kammerwinkel entsprechende Abschrägung 14 vorgesehen sein, die einen Winkel von zwischen 30° und 70°, vorzugsweise 37°, mit der Längsachse 15 des Katheters bildet.

## Patentansprüche

1. Vorrichtung zur Glaukombehandlung mit einem Laserkatheter und einer lichtführenden Faseranordnung, an deren proximalem Ende Licht einkoppelbar ist und an deren distalem Ende eine Lichtaustrittsfläche vorgesehen ist, **dadurch gekennzeichnet, dass** am distalen Ende des Laserkatheters (6) eine zur einfachen Ermittlung der richtigen Arbeitsposition bezüglich des Schlemm'schen Kanals (8) dienende Justiereinrichtung vorgesehen ist.

2. Vorrichtung nach Anspruch 1, **dadurch gekennzeichnet, dass** am distalen Ende des Laserkatheters (6) ein zur Abstützung auf der Iriswurzel (11) dienender Justiernocken (10) vorgesehen ist.

3. Vorrichtung nach Anspruch 1 oder 2, **dadurch gekennzeichnet, dass** der Justiernocken (10) an einem die Faseranordnung (7) umgebenden Stützmantel (9) angeformt ist.

4. Vorrichtung zur Glaukombehandlung mit einem Laserkatheter und einer lichtführenden Faseranordnung, an deren proximalem Ende Licht einkoppelbar ist und an deren distalem Ende eine Lichtaustrittsfläche vorgesehen ist, insbesondere nach einem der Ansprüche 1 bis 3, **gekennzeichnet durch** einen in den Schlemm'schen Kanal (8) einführbaren Stent (12) zur Ertastung der richtigen Arbeitsposition des Laserkatheters (6).

5. Vorrichtung nach Anspruch 4, **dadurch gekennzeichnet, dass** der Laserkatheter (6) am distalen Ende mit einer zur Form des Stents (12) komplementären Justierausnehmung (13) versehen ist.

6. Vorrichtung nach Anspruch 5, **dadurch gekennzeichnet, dass** die Justierausnehmung (13) in die Stirnfläche des Stützmantels (9) eingeformt ist.

7. Vorrichtung nach einem der Ansprüche 1 bis 6, **dadurch gekennzeichnet, dass** der Laserkatheter (6) am distalen Ende mit einer dem Kammerwinkel des Auges angepassten Abschrägung (14) versehen ist.

8. Vorrichtung nach Anspruch 7, **dadurch gekennzeichnet, dass** der Winkel der Abschrägung (14) zwischen 30° und 70°, vorzugsweise 37°, bezüglich der Längsachse (15) des Katheters beträgt.
